# EUROPÄISCHE PATENTSCHRIFT

(11) **EP 3 996 769 B1**
(45) Veröffentlichungstag und Bekanntmachung des Hinweises auf die Patenterteilung: **29.10.2025**
(21) Anmeldenummer: 20739946.0
(22) Anmeldetag: 09.07.2020
(51) Int. Cl.: A61M 1/16

(54) **DIALYSEGERÄT MIT ZWEI MITEINANDER IN FLUIDVERBINDUNG STEHENDEN BILANZKAMMERN**
DIALYSIS MACHINE WITH TWO BALANCE CHAMBERS THAT ARE IN FLUID COMMUNICATION WITH EACH OTHER
MACHINE DE DIALYSE AVEC DEUX CHAMBRES D'ÉQUILIBRE QUI SONT EN COMMUNICATION FLUIDIQUE L'UNE AVEC L'AUTRE

(30) Priorität: 09.07.2019 DE 102019118548
(43) Veröffentlichungstag der Anmeldung: 18.05.2022
(73) Patentinhaber: Fresenius Medical Care Deutschland GmbH, 61352 Bad Homburg (DE)
(72) Erfinder: IRRGANG, Tobias, 97633 Aubstadt (DE)
(74) Vertreter: Herrmann, Uwe
(86) Internationale Anmeldenummer: PCT/EP2020/069426
(87) Internationale Veröffentlichungsnummer: WO 2021/005173

(56) Entgegenhaltungen:
- DE-C1- 19 830 928
- US-A- 4 770 769

## Beschreibung

Die vorliegende Erfindung betrifft ein Dialysegerät gemäß dem Oberbegriff des Anspruchs 1.

Ein solches Gerät ist aus der US 4 770 769 A bekannt.

Das Dialysegerät umfasst einen Dialysator und eine erste Bilanzkammer A und eine zweite Bilanzkammer B, von denen jede wenigstens zwei durch eine bewegliche Wand voneinander getrennte erste und zweite Bilanzkammerhälften aufweist, wobei jede erste Bilanzkammerhälfte mit jeweils einem ersten Zulauf und mit jeweils einem ersten Ablauf versehen ist, wobei jede zweite Bilanzkammerhälfte mit jeweils einem zweiten Zulauf und mit jeweils einem zweiten Ablauf versehen ist, wobei die Zu- und die Abläufe jeweils mit Ventilen versehen sind, die ausgebildet sind, den jeweiligen Zulauf bzw. Ablauf zu schließen oder zu öffnen, wobei jeweils der erste Zulauf der ersten Bilanzkammerhälften mit einer Quelle frischen Dialysats und der jeweils erste Ablauf der ersten Bilanzkammerhälften mit einem Dialysatorzulauf in Fluidverbindung stehen und wobei jeweils der zweite Zulauf der zweiten Bilanzkammerhälften mit einem Dialysatorablauf in Fluidverbindung stehen.

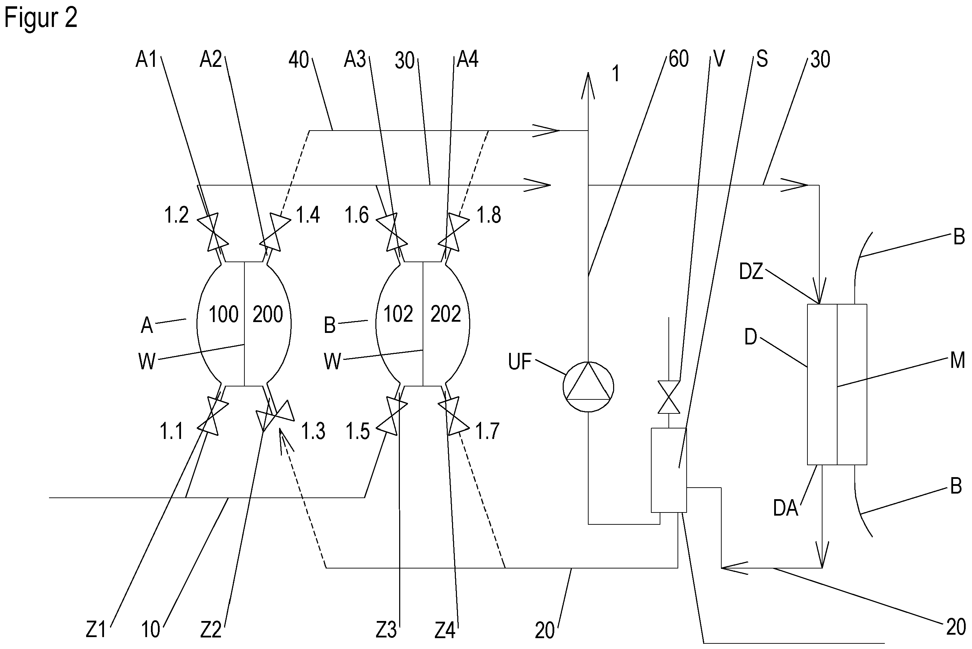

Ein derartiges Dialysegerät ist aus dem Stand der Technik bekannt und in Figur 2 exemplarisch dargestellt.

Figur 2 zeigt ein Dialysegerät im Zustand der "normalen" Hemodialyse ohne Substituatförderung.

Wie dies aus Figur 2 hervorgeht, sind zwei Bilanzkammern A und B vorgesehen, von denen jede eine erste Bilanzkammerhälfte 100, 102 und eine zweite Bilanzkammerhälfte 200, 202 aufweist. Die Bilanzkammerhälften sind jeweils durch eine bewegliche Wand W voneinander getrennt.

Aus Figur 2 ergibt sich des Weiteren, dass jede Bilanzkammerhälfte 100, 200, 102, 202 jeweils einen ersten Zulauf Z1 und Z3 für frisches Dialysat, das über die Leitung 10 zugeführt wird und jeweils einen zweiten Zulauf Z2 und Z4 für gebrauchtes Dialysat, das von dem Dialysator D über die Leitung 20 zugeführt wird, aufweist. Die Zuläufe Z1 und Z2 der ersten Bilanzkammer A sind mittels der Ventile 1.1 und 1.3 verschließbar, die Abläufe A1 und A2 der ersten Bilanzkammer A sind mittels der Ventile 1.2 und 1.4 verschließbar. Die Zuläufe Z3 und Z4 der zweiten Bilanzkammer B sind mittels der Ventile 1.5 und 1.7 verschließbar und die Abläufe A3 und A4 der zweiten Bilanzkammer B sind mittels der Ventile 1.6 und 1.8 verschließbar.

In den Figuren sind die Leitungen, in denen gebrauchtes Dialysat gefördert wird, gestrichelt dargestellt. Leitungen, in denen frisches Dialysat gefördert wird, sind mit durchgezogenen Linien dargestellt.

Die Leitung 30 steht mit den Abläufen A1 und A3 in Fluidverbindung und dient zur Förderung von frischem Dialysat zu dem Dialysator D bzw. zu dessen Zulauf DZ. Dieser ist durch die Membran M in zwei oder mehr als zwei Kammern unterteilt, von denen eine von Dialysat und die andere von Blut B durchströmt wird. Auf der Ablaufseite DA des Dialysators D gelangt das gebrauchte Dialysat einschließlich des Ultrafiltrats in den Sekundärluftabscheider S, der ein Ventil V zur Abfuhr von Luft aufweist.

Von dem Sekundärluftabscheider S wird ein Teil des gebrauchten Dialysats mittels der Ultrafiltrationspumpe UF mittels der Leitung 60 in den Drain 1 gefördert. Der verbleibende Teil des gebrauchten Dialysats gelangt getaktet über die Zuläufe Z2 und Z4 zu den zwei zweiten Bilanzkammerhälften 200, 202 der Bilanzkammern A und B.

Die Leitung 40 steht mit den Abläufen A2 und A4 der zweiten Bilanzkammerhälften 200, 202 in Fluidverbindung und dient zur Förderung von gebrauchtem Dialysat zu dem Drain 1.

Die bilanzierte Förderung des Dialysats gestaltet sich gemäß Figur 2 wie folgt:
Zunächst wird in die erste Bilanzkammerhälfte 100 der ersten Bilanzkammer A über die Leitung 10 Frischdialysat gefördert. Dazu ist das Ventil 1.1 geöffnet und das Ventil 1.2 ist geschlossen. Durch die sich dabei nach rechts bewegende Wand W wird gleichzeitig gebrauchtes Dialysat aus der zweiten Bilanzkammerhälfte 200 der ersten Bilanzkammer A über die Leitung 40 in den Drain 1 entfernt. Dabei ist das Ventil 1.3 geschlossen und das Ventil 1.4 ist geöffnet. Es wird somit genau ein Bilanzkammervolumen gefördert, beispielsweise 30 ml.

Die zweite Bilanzkammer B arbeitet gegengetaktet, d.h. Ventil 1.5 ist geschlossen, Ventil 1.6 ist geöffnet, Ventil 1.7 ist geöffnet und Ventil 1.8 ist geschlossen. Insgesamt wird durch die erste Bilanzkammer A in diesem Takt gebrauchtes Dialysat aus der ersten Bilanzkammer A gefördert, und aus der zweiten Bilanzkammer B Frischdialysat.

Im Anschluss daran erfolgt ein neuer Takt des Bilanzkammersystems, bei dem frisches Dialysat aus der ersten Bilanzkammerhälfte 100 der ersten Bilanzkammer A und gebrauchtes Dialysat aus der zweiten Bilanzkammerhälfte 202 der zweiten Bilanzkammer B gefördert wird.

Der oben beschriebene Ablauf wiederholt sich in stetiger Abfolge.

Es wird stets dieselbe Menge Frischdialysat und gebrauchtes Dialysat gefördert und exakt bilanziert.

Aufgrund der Entwässerung des Patienten entsteht auf der Seite des gebrauchten Dialysates zusätzlich Ultrafiltrat, das durch die Pumpe UF gefördert wird, die sich in der Leitung 60 befindet.

Bei einer Behandlung entstehen ca. 2l Ultrafiltrat pro Patient und einer Behandlungsdauer von ca. 4h.

Die Ultrafiltratmenge wird über die Ultrafiltratpumpe UF gesteuert, die das Ultrafiltrat aus dem Blut des Patienten abzieht.

Die aus dem Stand der Technik bekannte Anordnung gemäß Figur 2 sorgt für eine exakte Kontrolle der Dialysatflüssigkeitsmengen (frisch und gebraucht) und der Ultrafiltratmenge. Die Steuerung der Ultrafiltratmenge ist wichtig, weil eine Mindestmenge zur Vermeidung einer Überwässerung des Patienten gewährleistet sein muss und andererseits eine zu schnelle Entwässerung zu einem Kreislaufkollaps führen kann.

Gemäß dem Stand der Technik kann vorgesehen sein, dass eine zusätzliche Dialysatmenge bzw. Flüssigkeitsmenge über eine separate, nicht dargestellte Pumpe (Substituatpumpe) zum Patienten gefördert wird. Dies wird erforderlich, wenn eine größere Menge an Ultrafiltrat dem Blut entnommen wird als dem Patienten verschrieben wurde. Dann kann das Substituat (in der Regel) nach dem Dialysator zugeführt werden. Grundsätzlich ist auch Zugabe vor oder vor und nach dem Dialysator möglich. Substituatmengen zwischen 20 und 40 l pro Behandlungszyklus sind üblich.

Dieses Verfahren wird HDF (Hemodiafiltration) genannt. Der HDF Modus hat den Vorteil, dass ein erhöhter konvektiver Anteil über die Dialysatormembran befördert werden kann und somit insbesondere die mittelmolekularen Urämtoxine besonders effektiv entfernt werden können, die diffusiv nur im geringen Maße über die Membran hinweg befördert werden können.

Zusätzlich zur Substituatpumpe ist bei bekannten Geräten ein weiterer Filter für das Substituat erforderlich, welcher eine Keimfreifiltration des Substitutes sicherstellt. Dies ist deshalb erforderlich, weil das Substituat direkt in das Patientenblut infundiert wird.

Eine solche Anordnung ist somit kompliziert und teuer.

Der vorliegenden Erfindung liegt somit die Aufgabe zugrunde, ein Dialysegerät der eingangs genannten Art dahingehend weiterzubilden, dass dieses einen möglichst einfachen Aufbau aufweist.

Diese Aufgabe wird durch ein Dialysegerät mit den Merkmalen des Anspruchs 1 gelöst. Danach ist vorgesehen, dass die zweiten Abläufe der zweiten Bilanzkammerhälften miteinander in Fluidverbindung stehen, so dass gebrauchtes Dialysat von einer zweiten Bilanzkammerhälfte einer Bilanzkammer zu einer zweiten Bilanzkammerhälfte der anderen Bilanzkammer überführbar ist. Erfindungsgemäß ist somit vorgesehen, dass die Abläufe der zweiten Bilanzkammerhälften vorzugsweise direkt miteinander verbunden sind und ein Volumen (Pendelvolumen) von einer zweiten Bilanzkammerhälfte einer Bilanzkammer zu einer zweiten Bilanzkammerhälfte der anderen Bilanzkammer gefördert wird.

Es wird somit nicht bei dem Bilanzkammerzylus gebrauchtes Dialysat in den Ablauf gefördert, vielmehr dient eine bestimmte Menge des gebrauchten Dialysats als Pendelvolumen, das von einer Bilanzkammer zu der anderen Bilanzkammer geleitet wird. Dieser Vorgang kann sich einmal oder mehrfach vollziehen, d.h. das Volumen kann auch hin und her pendeln.

Da in diesem Fall kein gebrauchtes Dialysat aus dem System entfernt wird, wie dies gemäß dem Stand der Technik jedoch der Fall ist, jedoch weiterhin frisches Dialysat zugegeben wird, erhöht sich die Flüssigkeitsmenge im System. Diese Flüssigkeitsmenge wird über die Membran M des Dialysators D hinweg in den Patienten gefördert und gelangt so in das Blut B.

Diese Flüssigkeitsmenge (z.B. 1 Hub, 30 ml) dient als Substituat für den Patienten. Gleichzeitig wirkt die Membran M des Dialysators D als Sterilfilter für das Substiuat, so dass sowohl eine Substiuatpumpe als auch ein Sterilfilter obsolet sind. Abgesehen davon wird kein eigener Schlauchsatz für das Substituat benötigt.

Vorzugsweise ist vorgesehen, dass die zweiten Abläufe der zweiten Bilanzkammerhälften mit einer zu einem Abfluss führenden Drainleitung in Fluidverbindung stehen, in der ein Drainventil angeordnet ist, mittels derer die Drainleitung geöffnet und geschlossen werden kann. Während der oben beschriebenen Pendelbewegung ist vorzugsweise vorgesehen, dass dieses Drainventil geschlossen ist.

Erfindungsgemäß ist eine Steuereinheit vorgesehen, die ausgebildet ist, die Ventile so anzusteuern, dass bei geöffnetem ersten Zulauf und geschlossenem ersten Ablauf der ersten Bilanzkammerhälfte der ersten Bilanzkammer diese erste Bilanzkammerhälfte mit frischen Dialysat gefüllt wird, dass bei diesem Vorgang bei geschlossenem zweiten Zulauf und geöffnetem zweiten Ablauf die zweite Bilanzkammerhälfte dieser ersten Bilanzkammer entleert wird, wobei der zweite Ablauf der zweiten Bilanzkammerhälfte geöffnet ist, so dass das gebrauchte Dialysat von der zweiten Bilanzkammerhälfte der ersten Bilanzkammer in die zweite Bilanzkammerhälfte der zweiten Bilanzkammer gefördert wird, wobei der erste Ablauf der zweiten Bilanzkammerhälfte der zweiten Bilanzkammer geöffnet und der erste Zulauf der zweiten Bilanzkammerhälfte der zweiten Bilanzkammer geschlossen ist, so dass aus der zweiten Bilanzkammerhälfte frisches Dialysat zum Dialysator gefördert wird.

Der Vorgang gilt selbstverständlich auch umgekehrt, d.h. für eine Förderung des Pendelvolumens von der zweiten Bilanzkammer zu der ersten Bilanzkammer.

Vorzugsweise ist vorgesehen, dass das Gerät keine Substituatpumpe zur Förderung einer Substitutionslösung in das Blut des Patienten aufweist.

Wie ausgeführt, kann eine Steuerung vorgesehen sein, die ausgebildet ist, das Drainventil zu schließen, wenn gebrauchtes Dialysat von einer zweiten Bilanzkammerhälfte einer Bilanzkammer zu einer zweiten Bilanzkammerhälfte der anderen Bilanzkammer überführt wird.

Des Weiteren kann eine Steuerung vorgesehen sein, die ausgebildet ist, die Ventile so anzusteuern, dass gebrauchtes Dialysat von einer zweiten Bilanzkammerhälfte einer Bilanzkammer zu einer zweiten Bilanzkammerhälfte der anderen Bilanzkammer überführt wird, und dass nach dieser Überführung oder nach mehreren derartigen Überführungen die zweiten Abläufe der zweiten Bilanzkammerhälften mit einem Abfluss für gebrauchtes Dialysat in Fluidverbindung gebracht werden.

Nach einer oder mehreren Hin- und Herförderungen des Pendelvolumens ist in diesem Fall vorgesehen, dass das gebrauchte Dialysat dem Drain zugeführt wird.

Denkbar ist es, dass eine Steuerung vorgesehen ist, die ausgebildet ist, die Überführung gebrauchten Dialysats, d.h. des Pendelvolumens zu jedem n-ten Takt des Betriebs des Bilanzkammersystems durchzuführen. In diesem Fall erfolgt die Pendelbewegung z.B. zu jedem 10. Takt des Bilanzkammersystems.

Denkbar ist es weiterhin, dass eine Steuerung vorgesehen ist, die ausgebildet ist, die Überführung gebrauchten Dialysats von der zweiten Bilanzkammerhälfte einer Bilanzkammer zu der zweiten Bilanzkammerhälfte der anderen Bilanzkammer über die Behandlungsdauer zeitlich gleichmäßig oder zeitlich ungleichmäßig verteilt durchzuführen. Zu welchen Zeitpunkten die Überführung des Pendelvolumens, d.h. die Pendelbewegung erfolgt, kann beispielsweise von einer ärztlichen Vorschrift abhängig sein.

Vorzugsweise handelt es sich bei dem Dialysator um einen High-Flux-Dialysator oder um einen Mid-cut-off-Dialysator. Als High- Flux Dialysatoren werden Filter bezeichnet, die eine Ultrafiltrationrate von 20-70 mL/ m2 * mmHg * h in Humanblut aufweisen. Bei diesen Dialysatoren ist die erfindungsgemäße Substituatzuführung aufgrund der hohen Wasserpermeabilität in Vollblut besonders leicht zu erzielen. Dabei werden bevorzugt Substituatmengen von 5 bis 25 l pro 4-stündiger Behandlung eingestellt, besonders bevorzugt 15 bis 25 I pro 4-stündiger Behandlung.

Besonders effizient wirkt das erfindungsgemäße Dialysegerät bei Mid-cut-off- oder "Protein-leaking"- Dialysatoren. Solche Dialysatoren weisen eine noch höhere Ultrafiltrationsrate in Vollblut auf als High-Flux- Dialysatoren, als nachteilig erweist sich jedoch der erhöhte Albuminverlust, der bis zu 8 g bei einer Behandlung von 4 Stunden betragen kann. Bei Mid-cut- off Dialysatoren findet eine Substituatzuführung durch unkontrollierte Rückfiltration statt. Mit einer erfindungsgemäßen Dialysemaschine kann die Substituatmenge exakt gesteuert werden. Insbesondere sind auch höhere Substituatmengen möglich als mit einer Dialysemaschine gemäß dem Stand der Technik, die mit einem Mid-Cut-off- Dialysator betrieben wird. So kann die optimale Substituatmenge unter Berücksichtigung des erlaubten Albuminverlustes auch bei unterschiedlichen Hämatokritwerten des Patientenblutes bereitgestellt werden. Erfindungsgemäß können Substituatmengen von 5-20 I bei einer 4-stündigen HD-Behandlung eingestellt werden. Bevorzugt sind Substituatmengen von 8 bis 15 l pro 4-stündiger Behandlung.

Die vorliegende Erfindung betrifft des Weiteren ein Verfahren zum Betreiben eines Bilanzkammersystems mit einer ersten Bilanzkammer und mit einer zweiten Bilanzkammer, von denen jede wenigstens zwei durch eine bewegliche Wand voneinander getrennte erste und zweite Bilanzkammerhälften aufweist, wobei jede erste Bilanzkammerhälfte mit jeweils einem ersten Zulauf und mit jeweils einem ersten Ablauf versehen ist, wobei jede zweite Bilanzkammerhälfte mit jeweils einem zweiten Zulauf und mit jeweils einem zweiten Ablauf versehen ist, wobei die Zu- und die Abläufe jeweils mit Ventilen versehen sind, die ausgebildet sind, den jeweiligen Zulauf bzw. Ablauf zu schließen oder zu öffnen, wobei der erste Zulauf der ersten Bilanzkammerhälften mit einer Quelle frischen Dialysats und der erste Ablauf der ersten Bilanzkammerhälften mit einem Dialysatorzulauf in Fluidverbindung stehen und wobei der zweite Zulauf der zweiten Bilanzkammerhälften jeweils mit einem Dialysatorablauf in Fluidverbindung steht, wobei in einem ersten Betriebsmodus gebrauchtes Dialysat von der zweiten Bilanzkammerhälfte der ersten Bilanzkammer zu der zweiten Bilanzkammerhälfte der zweiten Bilanzkammer überführt wird, während die erste Bilanzkammerhälfte der ersten Bilanzkammer mit frischem Dialysat gefüllt wird und gebrauchtes Dialysat aus der zweiten Bilanzkammerhälfte der zweiten Bilanzkammer entleert wird.

In dem ersten Betriebsmodus kann gebrauchtes Dialysat auch von der zweiten Bilanzkammerhälfte der zweiten Bilanzkammer zu der zweiten Bilanzkammerhälfte der ersten Bilanzkammer überführt werden, während die erste Bilanzkammerhälfte der zweiten Bilanzkammer mit frischem Dialysat gefüllt wird und aus der zweiten Bilanzkammerhälfte der ersten Bilanzkammer gebrauchtes Dialysat entleert wird.

Die Überführung des Pendelvolumens kann somit von der ersten zur zweiten Bilanzkammer und auch umgekehrt von der zweiten zur ersten Bilanzkammer erfolgen.

Die Überführung des Pendelvolumens von einer zu der anderen Bilanzkammer kann einmal oder mehrmals erfolgen.

Die Überführung kann mehrmals unmittelbar hintereinander oder mit dazwischenliegenden Takten der Bilanzkammer ohne Überführung erfolgen.

Auch ist es denkbar, dass in einem zweiten Betriebsmodus gebrauchtes Dialysat von jeder der zweiten Bilanzkammerhälften in den Abfluss gefördert wird. In diesem Fall existiert kein Pendelvolumen und das Dialysegerät wird betrieben, wie dies aus dem Stand der Technik bekannt ist.

Wie ausgeführt ist es von Vorteil, wenn zur Zufuhr von Substituat zum Patienten keine Substituatpumpe verwendet wird. Auf eine solche kann verzichtet werden, da die überschüssige Menge an frischem Dialysat über die Membran des Dialysators dem Patienten zugeführt wird.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: eine schematische Ansicht des Dialysatkreislaufes eines Dialysegerätes gemäß der Erfindung und
- Figur 2:: eine schematische Ansicht des Dialysatkreislaufes eines Dialysegerätes gemäß dem Stand der Technik.

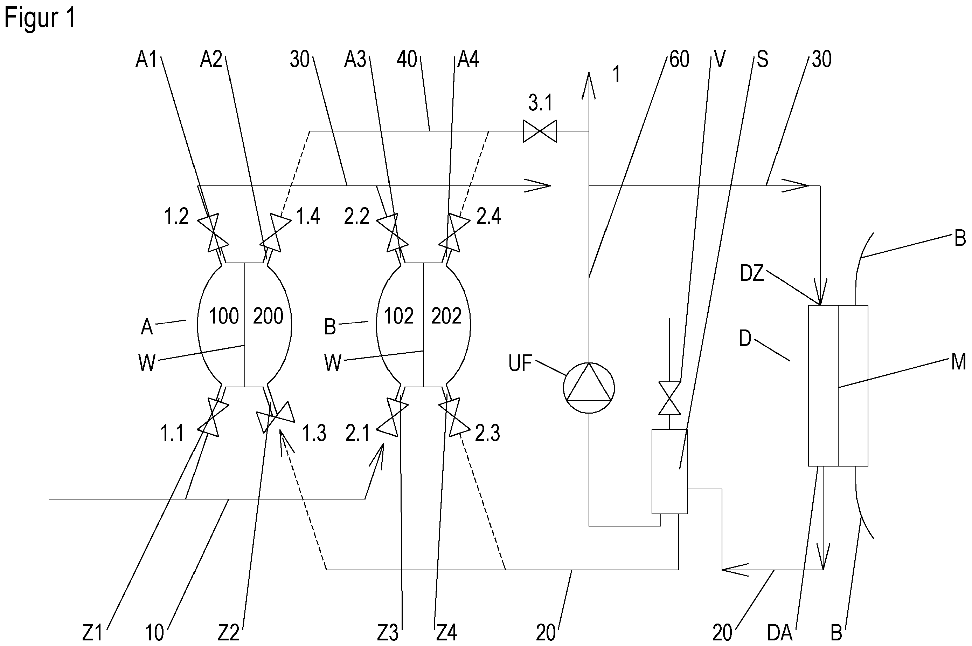

In Figur 1 sind gleiche oder funktionsgleiche Elemente mit denselben Bezugszeichen gekennzeichnet wie in Figur 2, so dass entsprechend Bezug genommen wird.

Der Betrieb des erfindungsgemäßen Dialysegeräts gemäß Figur 1 gestaltet sich wie folgt.

Zunächst wird die erste Bilanzkammerhälfte 100 der ersten Bilanzkammer A wiederum mit Frischdialysat befüllt, V1.1 ist geöffnet und V1.2 geschlossen, V1.3 geschlossen und V1.4 geöffnet. Die erste Bilanzkammerhälfte 100 wird somit mit Frischdialysat befüllt und gebrauchtes Dialysat wird aus zweiten Bilanzkammerhälfte 200 der ersten Bilanzkammer A entfernt.

In der zweiten Bilanzkammer B ist wiederum ein gegenläufiger Takt eingerichtet.

Es wird Frischdialysat aus der Bilanzkammerhälfte 102 entfernt, wobei Ventil V2.1 geschlossen und V2.2 geöffnet ist. Nunmehr ist Ventil V2.4 geöffnet, V2.3 bleibt geschlossen. Nach Abschluss des Vorganges findet wiederum eine Umschaltung auf den Gegentakt statt.

Diese Schaltung führt dazu, dass gebrauchtes Dialysat zwischen den beiden Bilanzkammern bewegt wird bzw. hin und herbewegt wird.

Damit wird in diesem Betriebsmodus kein gebrauchtes Dialysat mehr aus dem System entfernt, wie in der Situation von Figur 2 der Fall ist. Da jedoch weiter frisches Dialysat in das System gefördert wird, erhöht sich die Flüssigkeitsmenge. Diese Flüssigkeitsmenge wird über die Membran des Dialysators hinweg in den Patienten gefördert, womit diese Flüssigkeitsmenge (mindestens 1 Hub, beispielsweise 30 ml), als Substituat für den Patienten wirkt.

Gleichzeitig wirkt die Dialysatormembran als Sterilfilter für das Susbstituat. Es wird somit ein interner HDF- Modus bereitgestellt, so dass auf einen eigens dafür vorgesehenen Sterilfilter verzichtet wird.

Es besteht der Vorteil, dass keine eigene Substituatpumpe, kein separater Schlauchsatz und kein separater Sterilfilter bereitgestellt werden müssen.

Während dieser Pendelbewegung des gebrauchten Dialysates von einer zweiten Bilanzkammerhälfte zur anderen und/oder umgekehrt kann vorgesehen sein, dass ein Ventil 3.1 in der Leitung 40 stromabwärts der Bilanzkammern geschlossen ist.

Es kann vorgesehen sein (bevorzugt), dass nur eine Füllmenge einer Bilanzkammer verwendet wird, um ein Substituat bereitzustellen (also beispielsweise 30 ml).

Es kann auch vorgesehen sein, dass ein "Doppelhub durchgeführt wird, d.h. eine Menge an gebrauchtem Dialysat also einmal "hin" und einmal "her" geschoben wird.

Es kann vorgesehen sein, dass beispielsweise jeder 10. Takt benutzt wird, um Substituat zu erzeugen.

Die Vorrichtung kann eine Auswerte- und Steuereinheit umfassen, die die ärztlich verschriebene Substituatmenge gleichmäßig auf die Behandlungsdauer verteilt.

Die Vorrichtung kann eine Auswerte- und Steuereinheit umfassen, die die ärztlich verschriebene Substituatmenge ungleichmäßig auf die Behandlungsdauer verteilt. Das heißt, dass beispielsweise die Substituatmenge am Anfang der Behandlung höher oder geringer ist als am Behandlungsende.

Die Vorrichtung wirkt besonders gut, wenn Dialysatoren verwendet werden, die als High- Flux Dialysatoren bezeichnet werden. Als High- Flux Dialysatoren werden Filter bezeichnet, die eine Ultrafiltrationrate von 20-70 ml/ m2 * mmHg * h in Humanblut aufweisen.

Besonders wirkt die erfindungsgemäße Vorrichtung in Verbindung mit einem sog. Mid-cut-off Dialysator. Ein solcher Dialysator ist beispielsweise in der WO 2015/118046 A beschrieben, auf die hier insoweit Bezug genommen wird. Solche Dialysatoren weisen eine unkontrollierte Rückspülung von Dialysat in den Blutkreislauf auf, was durch die internen Druckverhältnisse im Dialysator zustande kommt. Diese Dialysatoren dürfen nicht im HDF- Modus betrieben werden, da ansonsten der Verlust an Albumin bedeutend zu hoch ausfallen würde.

Mit Hilfe der erfindungsgemäßen Vorrichtung kann nun mit einer "normalen" Maschine im "kontrollierten Substituatmodus" betrieben werden. Insbesondere kann durch geschickte Programmwahl die Substituatmenge auf Volumina zwischen 2 und 15 l / Behandlung, bevorzugt auf Volumina zwischen 5 und 12 l / Behandlung, weiter bevorzugt auf Volumina zwischen 5 und 10 l / Behandlung begrenzt werden.

## Patentansprüche

1. Dialysegerät mit einem Dialysator (D) und mit einer ersten Bilanzkammer (A) und mit einer zweiten Bilanzkammer (B), von denen jede wenigstens zwei durch eine bewegliche Wand (W) voneinander getrennte erste (100, 102) und zweite Bilanzkammerhälften (200, 202) aufweist, wobei jede erste Bilanzkammerhälfte (100, 102) mit jeweils einem ersten Zulauf (Z1, Z3) und mit jeweils einem ersten Ablauf (A1, A3) versehen ist, wobei jede zweite Bilanzkammerhälfte (200, 202) mit jeweils einem zweiten Zulauf (Z2, Z4) und mit jeweils einem zweiten Ablauf (A2, A4) versehen ist, wobei die Zu- und die Abläufe (A1-A4, Z1-Z4) jeweils mit Ventilen (1.1-1.4; 2.1-2.4) versehen sind, die ausgebildet sind, den jeweiligen Zulauf (Z1-Z4) bzw. Ablauf (A1-A4) zu schließen oder zu öffnen, wobei die ersten Zuläufe (Z1; Z3) der ersten Bilanzkammerhälften (100; 102) mit einer Quelle frischen Dialysats und der erste Ablauf (A1) der ersten Bilanzkammerhälfte (100) mit einem Dialysatorzulauf in Fluidverbindung steht und wobei die zweiten Zuläufe (Z2; Z4) der zweiten Bilanzkammerhälften (200; 202) mit einem Dialysatorablauf in Fluidverbindung stehen, wobei die zweiten Abläufe (A2; A4) der zweiten Bilanzkammerhälften (200, 202) miteinander in Fluidverbindung stehen, so dass gebrauchtes Dialysat von einer zweiten Bilanzkammerhälfte (200) einer Bilanzkammer (A) zu einer zweiten Bilanzkammerhälfte (202) der anderen Bilanzkammer (B) überführbar ist, **dadurch gekennzeichnet, dass** eine Steuereinheit vorgesehen ist, die ausgebildet ist, die Ventile (1.1-1.4; 2.1-2.4) so anzusteuern, dass bei geöffnetem ersten Zulauf (Z1) und geschlossenem ersten Ablauf (A1) der ersten Bilanzkammerhälfte (100) einer Bilanzkammer (A) diese erste Bilanzkammerhälfte (100) mit frischen Dialysat gefüllt wird, dass bei diesem Vorgang bei geschlossenem zweiten Zulauf (Z2) und geöffnetem zweiten Ablauf (A2) die zweite Bilanzkammerhälfte (200) der ersten Bilanzkammer (A) entleert wird, wobei der zweite Ablauf (A2) der zweiten Bilanzkammerhälfte (200) geöffnet ist, so dass das gebrauchte Dialysat von der zweiten Bilanzkammerhälfte (200) der ersten Bilanzkammer (A) in die zweite Bilanzkammerhälfte (202) der zweiten Bilanzkammer (B) gefördert wird, wobei der erste Ablauf (A3) der ersten Bilanzkammerhälfte (102) der zweiten Bilanzkammer (B) geöffnet und der erste Zulauf (Z3) der ersten Bilanzkammerhälfte (102) der zweiten Bilanzkammer (B) geschlossen ist, so dass aus der zweiten Bilanzkammerhälfte (102) der zweiten Bilanzkammer (B) frisches Dialysat zum Dialysator (D) gefördert wird..

2. Dialysegerät nach Anspruch 1, **dadurch gekennzeichnet, dass** die zweiten Abläufe (A2; A4) der zweiten Bilanzkammerhälften (200; 202) mit einer zu einem Abfluss führenden Drainleitung (40) in Fluidverbindung stehen, in der ein Drainventil (3.1) angeordnet ist, mittels derer die Drainleitung (40) geöffnet und geschlossen werden kann.

3. Dialysegerät nach einem der vorhergehenden Ansprüche, **dadurch gekennzeichnet, dass** das Gerät keine Substituatpumpe zur Förderung einer Substitutionslösung in das Blut des Patienten aufweist.

4. Dialysegerät nach einem der Ansprüche 2 oder 3, **dadurch gekennzeichnet, dass** eine Steuerung vorgesehen ist, die ausgebildet ist, das Drainventil (3.1) zu schließen, wenn gebrauchtes Dialysat von einer zweiten Bilanzkammerhälfte (200; 202) einer Bilanzkammer (A; B) zu einer zweiten Bilanzkammerhälfte (200; 202) der anderen Bilanzkammer (A; B) überführt wird.

5. Dialysegerät nach einem der vorhergehenden Ansprüche, **dadurch gekennzeichnet, dass** eine Steuerung vorgesehen ist, die ausgebildet ist, die Ventile (1.1-1.4; 2.1-2.4) so anzusteuern, dass gebrauchtes Dialysat von einer zweiten Bilanzkammerhälfte (200; 202) einer Bilanzkammer (A; B) zu einer zweiten Bilanzkammerhälfte (200; 202) der anderen Bilanzkammer (A; B) überführt wird, und dass nach dieser Überführung oder nach mehreren derartigen Überführungen die zweiten Abläufe (A2; A4) der zweiten Bilanzkammerhälften (200; 202) mit einem Abfluss (1) für gebrauchtes Dialysat in Fluidverbindung gebracht werden.

6. Dialysegerät nach Anspruch 5, **dadurch gekennzeichnet, dass** eine Steuerung vorgesehen ist, die ausgebildet ist, die Überführung gebrauchten Dialysats von der zweiten Bilanzkammerhälfte (200; 202) einer Bilanzkammer (A; B) zu der zweiten Bilanzkammerhälfte (200; 202) der anderen Bilanzkammer (A; B) zu jedem n-ten Takt des Betriebs des Bilanzkammersystems durchzuführen.

7. Dialysegerät nach einem der vorhergehenden Ansprüche, **dadurch gekennzeichnet, dass** eine Steuerung vorgesehen ist, die ausgebildet ist, die Überführung gebrauchten Dialysats von der zweiten Bilanzkammerhälfte (200; 202) einer Bilanzkammer (A; B) zu der zweiten Bilanzkammerhälfte (200; 202) der anderen Bilanzkammer (A; B) über die Behandlungsdauer zeitlich gleichmäßig oder zeitlich ungleichmäßig verteilt durchzuführen.

8. Dialysegerät nach einem der vorhergehenden Ansprüche, **dadurch gekennzeichnet, dass** es sich bei dem Dialysator (D) um einen High-Flux-Dialysator oder um einen Mid-cut-off-Dialysator handelt.

9. Dialysegerät konfiguriert zur Durchführung eines Verfahrens zum Betreiben eines Bilanzkammersystems des Dialysegerätes mit einer ersten Bilanzkammer (A) und mit einer zweiten Bilanzkammer (B), von denen jede wenigstens zwei durch eine bewegliche Wand (W) voneinander getrennte erste (100, 102) und zweite Bilanzkammerhälften (200, 202) aufweist, wobei jede erste Bilanzkammerhälfte (100, 102) mit jeweils einem ersten Zulauf (Z1, Z3) und mit jeweils einem ersten Ablauf (A1, A3) versehen ist, wobei jede zweite Bilanzkammerhälfte (200, 202) mit jeweils einem zweiten Zulauf (Z2, Z4) und mit jeweils einem zweiten Ablauf (A2, A4) versehen ist, wobei die Zu- und die Abläufe (A1-A4, Z1-Z4) jeweils mit Ventilen (1.1-1.4; 2.1-2.4) versehen sind, die ausgebildet sind, den jeweiligen Zulauf (Z1-Z4) bzw. Ablauf (A1-A4) zu schließen oder zu öffnen, wobei die ersten Zuläufe (Z1; Z3) der ersten Bilanzkammerhälften (100; 102) mit einer Quelle frischen Dialysats und der erste Ablauf (A1) der ersten Bilanzkammerhälfte (100) mit einem Dialysatorzulauf in Fluidverbindung steht und wobei die zweiten Zuläufe (Z2; Z4) der zweiten Bilanzkammerhälften (200; 202) mit einem Dialysatorablauf in Fluidverbindung stehen, **dadurch gekennzeichnet, dass** in einem ersten Betriebsmodus gebrauchtes Dialysat von der zweiten Bilanzkammerhälfte (200) der ersten Bilanzkammer (A) zu der zweiten Bilanzkammerhälfte (202) der zweiten Bilanzkammer (B) überführt wird, während die erste Bilanzkammerhälfte (100) der ersten Bilanzkammer (A) mit frischem Dialysat gefüllt wird und frisches Dialysat aus der ersten Bilanzkammerhälfte (102) der zweiten Bilanzkammer (B) zum Dialysator (D) gefördert wird.

10. Dialysegerät nach Anspruch 9, **dadurch gekennzeichnet, dass** in dem ersten Betriebsmodus gebrauchtes Dialysat von der zweiten Bilanzkammerhälfte (202) der zweiten Bilanzkammer (B) zu der zweiten Bilanzkammerhälfte (200) der ersten Bilanzkammer (A) überführt wird, während die erste Bilanzkammerhälfte (102) der zweiten Bilanzkammer (B) mit frischem Dialysat gefüllt wird und aus der ersten Bilanzkammerhälfte (100) der ersten Bilanzkammer (A) frisches Dialysat zum Dialysator (D) gefördert wird.

11. Dialysegerät nach Anspruch 9 oder 10, **dadurch gekennzeichnet, dass** die Überführung einmal oder mehrmals erfolgt.

12. Dialysegerät nach Anspruch 11, **dadurch gekennzeichnet, dass** die Überführung mehrmals unmittelbar hintereinander oder mit dazwischenliegenden Takten der Bilanzkammer ohne Überführung erfolgt.

13. Dialysegerät nach einem der Ansprüche 9 bis 12, **dadurch gekennzeichnet, dass** in einem zweiten Betriebsmodus gebrauchtes Dialysat von jeder der zweiten Bilanzkammerhälften (200; 202) der Bilanzkammern (A; B) in den Abfluss (1) gefördert wird.

14. Dialysegerät nach einem der Ansprüche 9 bis 13, **dadurch gekennzeichnet, dass** zur Zufuhr von Substituat zum Patienten keine Substituatpumpe verwendet wird.

## Claims

1. Dialysis machine having a dialyzer (D) and having a first balancing chamber (A) and having a second balancing chamber (B) of which each comprises at least two first (100, 102) and second balancing chamber halves (200, 202) separated from one another by a movable wall (W), wherein each first balancing chamber half (100, 102) is provided with a respective first inflow (Z1, Z3) and with a respective first outflow (A1, A3), wherein each second balancing chamber half (200, 202) is provided with a respective second inflow (Z2, Z4) and with a respective second outflow (A2, A4), wherein the inflows and outflows (A1-A4, Z1-Z4) are each provided with valves (1.1-1.4; 2.1-2.4) that are configured to close or to open the respective inflow (Z1-Z4) or outflow (A1-A4), wherein the first inflows (Z1; Z3) of the first balancing chamber halves (100) are in fluid communication with a source of fresh dialysate and the first outflow (A1) of the first balancing chamber half (100) is in fluid communication with a dialyzer inflow, and wherein the second inflows (Z2; Z4) of the second balancing chamber halves (200; 202) are in fluid communication with a dialyzer outflow, wherein the second outflows (A2; A4) of the second balancing chamber halves (200, 202) are in fluid communication with one another so that consumed dialysate is transferrable from a second balancing chamber half (200) of a balancing chamber (A) to a second balancing chamber half (202) of the other balancing chamber (B), **characterized in that** a control unit is provided that is configured to control the valves (1.1-1.4; 2.1-2.4) such that with an open first inflow (Z1) and a closed first outflow (A1) of the first balancing chamber half (100) of a balancing chamber (A), this first balancing chamber half (100) is filled with fresh dialysate, such that in this process with a closed second inflow (Z2) and an open second outflow (A2), the second balancing chamber half (200) of the first balancing chamber (A) is emptied, wherein the second outflow (A2) of the second balancing chamber half (200) is opened so that the consumed dialysate is conveyed from the second balancing chamber half (200) of the first balancing chamber (A) into the second balancing chamber half (202) of the second balancing chamber (B), wherein the first outflow (A3) of the second balancing chamber half (102) of the second balancing chamber (B) are opened and the first inflow (Z3) of the first balancing chamber half (102) of the second balancing chamber (B) is closed so that fresh dialysate is conveyed from the second balancing chamber half (102) of the second balancing chamber (B) to the dialyzer (D).

2. Dialysis machine in accordance with claim 1, **characterized in that** the second outflows (A2, A4) of the second balancing chamber halves (200; 202) are in fluid communication with a drain line (40) that leads to an outlet, in which a drain valve (3.1) is arranged, and by means of which the drain line (40) can be opened and closed.

3. Dialysis machine in accordance with any one of the preceding claims, **characterized in that** the machine does not have any substituate pump for conveying a substitution solution into the blood of the patient.

4. Dialysis machine in accordance with one of the claims 2 or 3, **characterized in that** a control is provided that is configured to close the drain valve (3.1) when consumed dialysate is transferred from a second balancing chamber half (200; 202) of a balancing chamber (A; B) to a second balancing chamber half (200; 202) of the other balancing chamber (A; B).

5. Dialysis machine in accordance with any one of the preceding claims, **characterized in that** a control is provided that is configured to control the valves (1.1-1.4; 2.1-2.4) such that consumed dialysate is transferred from a second balancing chamber half (200; 202) of a balancing chamber (A; B) to a second balancing chamber half (200; 202) of the other balancing chamber (A; B) and such that after this transfer or after a plurality of such transfers, the second outflows (A2; A4) of the second balancing chamber halves (200; 202) are moved into fluid communication with an outlet (1) for consumed dialysate.

6. Dialysis machine in accordance with claim 5, **characterized in that** a control unit is provided that is configured to carry out the transfer of consumed dialysate from the second balancing chamber half (200; 202) of one balancing chamber (A; B) to the second balancing chamber half (200; 202) of the other balancing chamber (A; B) at each n^{th} cycle of the operation of the balancing chamber system.

7. Dialysis machine in accordance with any one of the preceding claims, **characterized in that** a control unit is provided that is configured to carry out the transfer of consumed dialysate from the second balancing chamber half (200; 202) of a balancing chamber (A; B) to the second balancing chamber half (200; 202) of the other balancing chamber (A; B) evenly in time or unevenly in time over the treatment duration.

8. Dialysis machine in accordance with any one of the preceding claims, **characterized in that** the dialyzer (D) is a high-flux dialyzer or a medium cut-off dialyzer.

9. Dialysis machine, configured for carrying out a method of operating a balancing chamber system of the dialysis machine having a first balancing chamber (A) and having a second balancing chamber (B) of which each comprises at least two first (100, 102) and second balancing chamber halves (200, 202) separated from one another by a movable wall (W), wherein each first balancing chamber half (100, 102) is provided with a respective first inflow (Z1, Z3) and with a respective first outflow (A1, A3), wherein each second balancing chamber half (200, 202) is provided with a respective second inflow (Z2, Z4) and with a respective second outflow (A2, A4), wherein the inflows and outflows (A1-A4, Z1-Z4) are each provided with valves (1.1-1.4; 2.1-2.4) that are configured to close or to open the respective inflow (Z1-Z4) or outflow (A1-A4), wherein the first inflows (Z1; Z3) of the first balancing chamber halves (100) are in fluid communication with a source of fresh dialysate and the first outflow (A1) of the first balancing chamber half (100) is in fluid communication with a dialyzer inflow, and wherein the second inflows (Z2; Z4) of the second balancing chamber halves (200; 202) are in fluid communication with a dialyzer outflow, **characterized in that** dialysate consumed in a first operating mode is transferred from the second balancing chamber half (200) of the first balancing chamber (A) to the second balancing chamber half (202) of the second balancing chamber (B), while the first balancing chamber half (100) of the first balancing chamber (A) is filled with fresh dialysate and fresh dialysate is conveyed from the first balancing chamber half (102) of the second balancing chamber (B) to the dialyzer (D).

10. Dialysis machine in accordance with claim 9, **characterized in that** dialysate consumed in the first operating mode is transferred from the second balancing chamber half (202) of the second balancing chamber (B) to the second balancing chamber half (200) of the first balancing chamber (A), while the first balancing chamber half (102) of the second balancing chamber (B) is filled with fresh dialysate and fresh dialysate is conveyed from the first balancing chamber half (100) of the first balancing chamber (A) to the dialyzer (D).

11. Dialysis machine in accordance with claim 9 or claim 10, **characterized in that** the transfer takes place once or a plurality of times.

12. Dialysis machine in accordance with claim 11, **characterized in that** the transfer takes place a plurality of times directly after one another or with interposed cycles of the balancing chamber without any transfer.

13. Dialysis machine in accordance with one of the claims 9 to 12, **characterized in that** dialysate consumed in a second operating mode is conveyed from each of the second balancing chamber halves (200; 202) of the balancing chambers (A; B) into the outlet (1).

14. Dialysis machine in accordance with one of the claims 9 to 13, **characterized in that** no substituate pump is used to supply substituate to the patient.

## Revendications

1. Appareil de dialyse avec un dialyseur (D) et avec une première chambre de bilan (A) et avec une deuxième chambre de bilan (B), dont chacune présente au moins deux premières (100, 102) et deuxièmes moitiés de chambre de bilan (200, 202) séparées l'une de l'autre par une paroi mobile (W), chaque première moitié de chambre de bilan (100, 102) étant pourvue respectivement d'une première entrée (Z1, Z3) et respectivement d'une première sortie (A1, A3), chaque deuxième moitié de chambre de bilan (200, 202) étant pourvue respectivement d'une deuxième entrée (Z2, Z4) et respectivement d'une deuxième sortie (A2, A4), les entrées et les sorties (A1-A4, Z1-Z4) étant respectivement pourvues de soupapes (1.1-1.4 ; 2.1-2.4) qui sont réalisées pour fermer ou pour ouvrir l'entrée (Z1-Z4) et la sortie (A1-A4) respectives, les premières entrées (Z1 ; Z3) des premières moitiés de chambres de bilan (100 ; 102) étant en communication fluidique avec une source de dialysat frais et la première sortie (A1) de la première moitié de chambre de bilan (100) étant en communication fluidique avec une entrée de dialyseur, et les deuxièmes entrées (Z2 ; Z4) des deuxièmes moitiés de chambres de bilan (200 ; 202) étant en communication fluidique avec une sortie de dialyseur, les deuxièmes sorties (A2 ; A4) des deuxièmes moitiés de chambres de bilan (200, 202) étant en communication fluidique l'une avec l'autre, de telle sorte que le dialysat usagé peut être transféré d'une deuxième moitié de chambre de bilan (200) d'une chambre de bilan (A) à une deuxième moitié de chambre de bilan (202) de l'autre chambre de bilan (B), **caractérisé en ce qu'**il est prévu une unité de commande qui est réalisée pour commander les soupapes (1.1-1.4 ; 2.1-2.4) de telle sorte que, lorsque la première entrée (Z1) est ouverte et la première sortie (A1) de la première moitié de chambre de bilan (100) d'une chambre de bilan (A) est fermée, cette première moitié de chambre de bilan (100) est remplie de dialysat frais, que, lors de ce processus, lorsque la deuxième entrée (Z2) est fermée et la deuxième sortie (A2) est ouverte, la deuxième moitié de chambre de bilan (200) de la première chambre de bilan (A) est vidée, la deuxième sortie (A2) de la deuxième moitié de chambre de bilan (200) étant ouverte, de telle sorte que le dialysat usagé est transporté de la deuxième moitié de chambre de bilan (200) de la première chambre de bilan (A) dans la deuxième moitié de chambre de bilan (202) de la deuxième chambre de bilan (B), la première sortie (A3) de la première moitié de chambre de bilan (102) de la deuxième chambre de bilan (B) étant ouverte et la première entrée (Z3) de la première moitié de chambre de bilan (102) de la deuxième chambre de bilan (B) étant fermée, de telle sorte que du dialysat frais est transporté de la deuxième moitié de chambre de bilan (102) de la deuxième chambre de bilan (B) au dialyseur (D).

2. Appareil de dialyse selon la revendication 1, **caractérisé en ce que** les deuxièmes sorties (A2 ; A4) des deuxièmes moitiés de chambres de bilan (200 ; 202) sont en communication fluidique avec une conduite de drainage (40) menant à une évacuation, dans laquelle est agencée une soupape de drainage (3.1), au moyen de laquelle la conduite de drainage (40) peut être ouverte et fermée.

3. Appareil de dialyse selon l'une quelconque des revendications précédentes, **caractérisé en ce que** l'appareil ne présente pas de pompe à substitut pour transporter une solution de substitution dans le sang du patient.

4. Appareil de dialyse selon l'une quelconque des revendications 2 ou 3, **caractérisé en ce qu'**il est prévu une commande qui est réalisée pour fermer la soupape de drainage (3.1) lorsque du dialysat usagé est transféré d'une deuxième moitié de chambre de bilan (200 ; 202) d'une chambre de bilan (A ; B) à une deuxième moitié de chambre de bilan (200 ; 202) de l'autre chambre de bilan (A ; B).

5. Appareil de dialyse selon l'une quelconque des revendications précédentes, **caractérisé en ce qu'**il est prévu une commande qui est réalisée pour commander les soupapes (1.1-1.4 ; 2.1-2.4) de telle sorte que du dialysat usagé soit transféré d'une deuxième moitié de chambre de bilan (200 ; 202) d'une chambre de bilan (A ; B) à une deuxième moitié de chambre de bilan (200 ; 202) de l'autre chambre de bilan (A ; B), et qu'après ce transfert ou après plusieurs tels transferts, les deuxièmes sorties (A2 ; A4) des deuxièmes moitiés de chambre de bilan (200 ; 202) soient mises en communication fluidique avec une évacuation (1) pour le dialysat usagé.

6. Appareil de dialyse selon la revendication 5, **caractérisé en ce qu'**il est prévu une commande qui est réalisée pour effectuer le transfert de dialysat usagé de la deuxième moitié de chambre de bilan (200 ; 202) d'une chambre de bilan (A ; B) à la deuxième moitié de chambre de bilan (200 ; 202) de l'autre chambre de bilan (A ; B) à chaque n-ième cycle du fonctionnement du système de chambres de bilan.

7. Appareil de dialyse selon l'une quelconque des revendications précédentes, **caractérisé en ce qu'**il est prévu une commande qui est réalisée pour effectuer le transfert de dialysat usagé de la deuxième moitié de chambre de bilan (200 ; 202) d'une chambre de bilan (A ; B) à la deuxième moitié de chambre de bilan (200 ; 202) de l'autre chambre de bilan (A ; B) de manière régulière dans le temps ou de manière irrégulière dans le temps pendant la durée du traitement.

8. Appareil de dialyse selon l'une quelconque des revendications précédentes, **caractérisé en ce que** le dialyseur (D) consiste en un dialyseur à haut débit ou un dialyseur à débit intermédiaire.

9. Appareil de dialyse configuré pour la mise en œuvre d'un procédé d'exploitation d'un système de chambres de bilan de l'appareil de dialyse avec une première chambre de bilan (A) et avec une deuxième chambre de bilan (B), dont chacune présente au moins deux premières (100, 102) et deuxièmes moitiés de chambre de bilan (200, 202) séparées l'une de l'autre par une paroi mobile (W), 202), chaque première moitié de chambre de bilan (100, 102) étant pourvue respectivement d'une première entrée (Z1, Z3) et respectivement d'une première sortie (A1, A3), chaque deuxième moitié de chambre de bilan (200, 202) étant pourvue respectivement d'une deuxième entrée (Z2, Z4) et respectivement d'une deuxième sortie (A2, A4), les entrées et les sorties (A1-A4, Z1-Z4) étant respectivement pourvues de soupapes (1.1-1.4 ; 2.1-2.4) qui sont réalisées pour fermer ou pour ouvrir l'entrée (Z1-Z4) et la sortie (A1-A4) respectives, les premières entrées (Z1 ; Z3) des premières moitiés de chambre de bilan (100 ; 102) étant en communication fluidique avec une source de dialysat frais et la première sortie (A1) de la première moitié de chambre de bilan (100) étant en communication fluidique avec une entrée de dialyseur et les deuxièmes entrées (Z2 ; Z4) des deuxièmes moitiés de chambre de bilan (200 ; 202) étant en communication fluidique avec une sortie de dialyseur, **caractérisé en ce que,** dans un premier mode de fonctionnement, du dialysat usagé est transféré de la deuxième moitié de chambre de bilan (200) de la première chambre de bilan (A) à la deuxième moitié de chambre de bilan (202) de la deuxième chambre de bilan (B), tandis que la première moitié de chambre de bilan (100) de la première chambre de bilan (A) est remplie de dialysat frais et que du dialysat frais est transporté de la première moitié de chambre de bilan (102) de la deuxième chambre de bilan (B) au dialyseur (D).

10. Appareil de dialyse selon la revendication 9, **caractérisé en ce que**, dans le premier mode de fonctionnement, du dialysat usagé est transféré de la deuxième moitié de chambre de bilan (202) de la deuxième chambre de bilan (B) à la deuxième moitié de chambre de bilan (200) de la première chambre de bilan (A), tandis que la première moitié de chambre de bilan (102) de la deuxième chambre de bilan (B) est remplie de dialysat frais et que du dialysat frais est transporté de la première moitié de chambre de bilan (100) de la première chambre de bilan (A) au dialyseur (D).

11. Appareil de dialyse selon la revendication 9 ou 10, **caractérisé en ce que** le transfert est effectué une fois ou plusieurs fois.

12. Appareil de dialyse selon la revendication 11, **caractérisé en ce que** le transfert est effectué plusieurs fois immédiatement les unes après les autres ou avec des cycles intermédiaires de la chambre de bilan sans transfert.

13. Appareil de dialyse selon l'une quelconque des revendications 9 à 12, **caractérisé en ce que**, dans un deuxième mode de fonctionnement, du dialysat usagé est transporté de chacune des deuxièmes moitiés (200 ; 202) de chambre bilan des chambres bilan (A ; B) vers l'évacuation (1).

14. Appareil de dialyse selon l'une quelconque des revendications 9 à 13, **caractérisé en ce qu'**aucune pompe à substitut n'est utilisée pour amener du substitut au patient.
